Europäisches Patentamt

European Patent Office

Office européen des brevets

(19)

(11) Publication number: **0 180 483**
A2

# EUROPEAN PATENT APPLICATION

(12)

(21) Application number: **85307952.3**

(22) Date of filing: **01.11.85**

(51) Int. Cl.⁴: **A 61 K 7/16, A 61 K 9/12**

(30) Priority: **02.11.84 CA 466927**

(43) Date of publication of application: **07.05.86**
**Bulletin 86/19**

(84) Designated Contracting States: **AT BE CH DE FR GB IT LI LU NL SE**

(71) Applicant: **RUTLAND CONSULTING GROUP LIMITED, 3200 Park Place 666 Burrard Street, Vancouver British Columbia (CA)**

(72) Inventor: **Roydhouse, Richard Helm, 4697 3rd Avenue, Vancouver British Columbia (CA)**

(74) Representative: **Allard, Susan Joyce et al, BOULT, WADE & TENNANT 27 Furnival street, London EC4A 1PQ (GB)**

(54) **Oral fine dispersions sprays and emulsions.**

(57) Fine dispersion spray and emulsion formulations are used as delivery agents for preferably oral hygiene, the pressurized agents being used as an oral spray or a mouth-rinse with water and comprising a potable mono-, di- or polyhydric alcohol or mixture thereof as non-aqueous carrier base, and a substantially water-insoluble surfactant such as lecithin, the agent being in the form of a stable solution or suspension which, when released from pressure and mixed with air or water, forms an unstable aerosol or hydrosol respectively which then achieves a more stable state by coating out on a surface, such as teeth or gums to loosen plaque and reduce adhesive forces.

- 1 -

ORAL FINE DISPERSIONS SPRAYS AND EMULSIONS

The present invention relates to compositions or delivery agents, in the form of sprays or emulsions, for the delivery of small concentrated amounts of effective constituents to a surface, and in particular to oral sprays and mouthrinses for the promotion of oral hygiene.

The carrier base of the delivery agent permits the use of water-insoluble components which have not been readily useable until the present invention. Small, concentrated amounts of these components can now be coated on mouth surfaces by a "contact transfer" phenomena which is hereinafter defined.

Since at least the 4th Century B.C. when Aristotle related soft adherent food-derived debris to dental disease, the cleaning of teeth has been a primary method in the prevention of dental disease. The concept implies that the diet chosen by humans differs from the so-called natural state of man, and that the micro-organisms that man is exposed to, and is host to, combining with this diet, produce a layer of adherent material to the surface of the tooth now generally called dental plaque.

This micro-environmental and microbial effect spreads over the teeth in sheltered areas, gaining its food supply from the host and creating around the plaque an environment of potential harm to the host. Thus in some, but not all circumstances, dental disease occurs about these living deposits on living teeth. The exact biochemical processes remain obscure, but epidemiologic data shows a relationship of high probability between plaque, and dental caries (the erosion and dissolution of dental enamel and dentine) and periodontal disease (the progressive deterioration of the membrane holding the teeth in place, producing pockets beside the root surfaces of the teeth in the gum or periodontal tissues). It is noteworthy that after the initial lesion, in both dental caries and periodontal disorders, the invasion as it were of the underlying tissue produces a nidus or host for the plaque organisms. This nidus is less exposed to the oral environment than was the initial plaque. Given that mechanical clearance of the initial plaque was inadequate, and its persistent presence led to the invasive effect, then after initiation and the formation of the nidus, mechanical removal or clearance by the previous mechanical methods will be less effective. As is readily seen, therefore at highest risk are those areas or regions sheltered from lips, tongues and mechanical devices such as toothbrushes. It is readily seen that after initiation in these protected regions, the disease process will proceed despite the best mechanical intervention.

The general approach to dental disease protection has been to use scrub brushes (toothbrushes) and soap (flavoured potable forms called toothpastes or dentifrices). The surprising lack of success of these methods is well documented in the dental literature. Toothbrushes, with or without detergent dentifrices, have never been shown to influence the incidence of dental caries. One major exception is the use of fluoride as a preventive agent for dental caries. The inclusion of a source of fluoride in both dentifrices and mouthrinses with equal success in prevention, suggests that its efficacy is related to an alteration in the dental enamel - making the mineral crystal structure either more resistant to solution or encouraging remineralization - or both. But such is not related to the toothbrush itself. Some evidence suggests

0180483

that fluoride alters the metabolism within plaque and also by lowering the surface energy of the exposed dental enamel surface decreases the accumulation of plaque.

In relation to periodontal disease the toothbrush is generally thought to reduce gingivitis, that is, the inflammation of the gum collars around the tooth. While it is true that most periodontal diseases, that is, development of pockets about teeth, begin as gingivitis, it remains uncertain that all gingivitis proceeds to become periodontal disease. Thus elimination or decrease in gingivitis does not always imply cessation of periodontal disease.

The use of dental floss, a simple string or thin band which is pulled between the teeth to dislodge food, does lead to a decrease of stagnation between teeth. However a special technique must be used by the individual to disturb and remove dental plaque (as opposed to food particles) between teeth. As previously noted, areas of stagnation are the most likely sites for both dental caries and periodontal disease, such being represented by the interdental area between teeth (where adjacent teeth contact each other), and around the junction of gum with tooth crown - or the gingival area.

Thus successful methods of prevention of dental disease have been limited to increased mechanical cleansing by either more and better directed toothcleaning or to the use of fluoride. Recently the use of mouthrinses with varying ingredients have been tried and a supposed reduction in dental plaque occurs with concomitant decrease in gingival irritation. The major ingredients of these mouthrinses are supposedly products such as essential oils and sanguinarine - a root extract.

It is now observed that, in the natural state of mankind as evidenced by anthropologic specimens, dental caries and destructive periodontal disease is not common. Thus the assumption can be made that saliva in and of itself is a non-caries producing fluid, and does not lead to periodontal disorders. It is not known whether dental plaque accumulated to any degree in ancient man but it must be accepted that either (a) it did not or (b) it was harmless. A variety of studies show that present peoples isolated from modern diets (Eskimos and aboriginals of many lands) begin to have dental caries and periodontal disease when introduced to modern diets. Thus while saliva may be neutral or probably even preventive, as it were, in these diseases, the effects of diet overwhelms such mechanisms. The inventor has also observed that mechanical cleaning and rinsing seem relatively ineffective. The soaps used in dentifrices are mainly "soluble in water, will function in acid or alkaline solution" (Newbrun, E., 1983, "Cariology", Williams and Wilkins, Baltimore) and as Newbrun continues "There is no evidence to indicate that detergents alone clinically can remove plaque". Such applies to most mouthrinses as well.

A review of the nature of the tooth surface, shows that the surface to which plaque attacks is not the mineral surface, Glantz in Leach, S.A. 1980 "Dental Plaque and Surface Interactions in the Oral Cavity", IRC Press Ltd., London, England, at page 49 writes "It is not likely that specific bonds are responsible for the primary adhesion of micro-organism to teeth. Rather under physiological condition, oral adhesive events are mainly monitored by the adhesive of the absorbed biological films and based on general types of physico-chemical relations". The biological film, he describes, is called the acquired pellicle which is defined by Newbrun as a posteruptive film of salivary proteins and glycoproteins upon which food deposits, "the dental plaque proper, food and microbiota, follow".

Scrubbing with bristles and soaps removes both plaque, food and probably patches of acquired pellicle. Such bald spots become re-coated within minutes. Glantz speaks of "high-speed" recovery, meaning within two seconds to two hours. This may only occur in regions reached by the bristles.

The mechanism of attachment of the micro- organisms is a matter of hypothesis. As suggested by J. Van Houte (Leach 1980 at p. 69) glycoproteins play a role, and possibly glycolipids. Electrostatic forces are also involved, and charged polymers are thought to act as bridges from micro-organisms to pellicle and to adjacent micro-organisms.

As indicated the adhesive bridging appears immune to water and water-based detergents.

The present invention now attempts to overcome these aforementioned inadequacies of conventional oral hygiene by using a carrier base which solubilizes active water-insoluble components and which provides for delivery of said components by "contact transfer" to a surface, preferably in the oral cavity, in small concentrated amounts.

Furthermore, the present invention attempts to reduce adhesion between teeth surfaces and micro-organisms in dental plaque or the acquired pellicle and to provide a surface which reduces adhesive bridging in the first instance.

The present invention now utilizes some of those natural agents present in saliva and many natural foodstuffs, the use of which have been restricted in the past because of the inadequate delivery systems.

Thus, according to one aspect of the present invention, there is provided a pressurized delivery agent for oral hygiene which comprises a potable mono-, di- or polyhydric alcohol or mixture thereof as non-aqueous carrier base, and a substantially water-insoluble surfactant, the agent being in the form of a stable solution or suspension which, when released from pressure and mixed with air or water, forms an unstable aerosol or hydrosol respectively which then achieves a more stable state by coating out on a surface with which it contacts. Preferably, the delivery agent further comprises a propellant gas partially dissolved therein.

According to another aspect of the present invention there is provided a method of oral hygiene comprising applying to an area of the mouth the pressurized delivery agent defined above.

Thus, fundamental to formulations of the present invention is the nature of the base material used to maintain the selected compounds in a stable homogeneous state until use is desired. Most of the desired compounds are relatively insoluble in water and previous usages depended heavily upon suspending agents and high viscosity base materials to prevent separation and aggregation of active materials during storage. As is the case in many chemical reactions, the physical state of potential reactants may determine the speed of the reaction due to such matters as surface tension, surface area and the physical nature of molecules. In this latter case, the long molecules of proteins, carbohydrates and lipids under suitable conditions may coil up or become extended, exposing as it were a lesser or greater molecular surface area. In adhesion and surface phenomena, of great importance is the ability of long molecules to orient upon surfaces or interfaces with lipophobic or lipophylic, hydrophobic or hydrophylic endgroups oriented in similar directions. Much of the surfactant behaviour of small quantities is related to this orienting ability. Thus thick or high viscosity pastes or emulsions are by nature a disadvantage. The flocculation or aggregation of such active dispersed molecules is related to the nature of separating forces and media (such as emulsifiers) and to the degree of agitation possible before use, and the passage of time. Attempts to provide active molecules of water-insoluble materials leads to such emulsification methods that diminish or eliminate the reactivity of the chosen active material, or to mixtures which have overall the reverse effects of what is intended. For example the usual toothpaste or dentifrice increases the viscosity of saliva rather than decreasing it.

The novel delivery agent of the present invention use as a carrier base a mixture of potable mono-, di- or preferably polyhydric alcohols and similar non-aqueous media. Such permits the manufacture in bulk of the desired components of any particular formulation, and its stable storage prior to final packaging or use. The carrier base provides solubilization of chosen materials, and such materials need not be soluble in water, nor do they need dispersing as coated colloidal or suspended particles (as in water which is conventionally used). The novel carrier base also facilitates or permits the use of the necessary flavouring of materials for oral usage.

Present preferred potable alcohols are the polyhydric alcohols such as glycerol, propylene glycol, dipropylene glycol and ethylene propylene glycol. However, ethanol with glycerol is also particularly preferred.

The dental literature generally discusses at considerable length the proteins and muco-proteins in saliva. However these contribute to plaque formation and considerable efforts to block such accumulation have been reported, but without practical success.

The salivary lipids, most of which are insoluble in water, are well described by Dirksen (Leach 1980). Their appearance in a gross fashion can be seen in and around the various areas of stagnation in teeth by placing freshly extracted teeth in a solution of the dye, Sudan Black, in propylene glycol, and rinsing with propylene glycol.

A feature of such lipids, especially the fatty acids (stearic, oleic, palmitic and myristic) and lecithin, are their surface-active nature. However many of these materials are by their very nature insoluble in water. Saliva is a complicated biologic medium and its exact nature remains unexplained. The appearance of these lipids in saliva is not an excretory waste-product mechanism, nor accident, but represents the evolution of a liquid capable of coating food for transmission down the oesophagus to the stomach. Saliva is a lubricant fluid which prevents cheeks, tongue and other soft tissues from sticking together, and allows lumps of food to be slid over the tongue and down into the oesophagus. Applicant has now recognized that while the saliva fluid has certain lubricant and surface coating activities, one by-product as it were, is the preservation of teeth in a watery environment.

Thus, in preferred embodiments, the novel formulation of the present invention enhances the lubricant, wetting behaviour of saliva, especially the lipid and lipid-like sections, by use of some chemicals that exist in the normal saliva plus variations of altered naturally occurring surface active agents.

Among the lipids is lecithin, properly known as phosphatidylcholine. It is lipotropic and generally soluble in alcohols, mineral oils, and fatty acids. Lecithin is substantially insoluble in water but in the presence of sodium chloride will form a colloidal suspension. In saliva 14 percent of the total lipid content is lecithin; lipids in themselves constitute from 2 to 7% of saliva depending on location of the supplying glands and on the nature of the flow. The total fatty acid content is about 40 to 50% of lipid content. Thus salivary lipids can be seen as colloidal agents within the protein-salt mix of saliva. As the lipids are insoluble in water, their presence suggests a function related to suspension or colloidal dispersion. Lecithin as a chemical lowers oil-water interfacial tension and allows emulsification to occur (Encyclopedia of Food Science Vol 3 1978 p 464); other valuable properties described is reduction of viscosity and increase in wetting as well as the preservation of emulsified fatty acids. Thus it can be hypothesized that some salivary lipids are held in colloidal emulsified form partly by the presence of lecithin.

A variety of theories have attempted to explain the adhesion of dental plaque and cohesion of microbiological masses. Glycoproteins and glycolipids may be involved (J. Van Houte, in Leach 1980 at p. 69), as will be electrostatic charges (Van der Walls forces, Edgar, M.M. et al, Leach 1980). Winter and others (in Leach 1980 at p. 211) suggest that calcium ions and lipoteichoc acid may contribute. Thus a complex mixture of systems are described, and probably these and unknown other system operate simultaneously.

The interaction of water and lipids with proteins and carbohydrates, such as occur in plaque and in stagnated areas, is of concern in the food industry where control of viscosity is desired. Hulton (Hulton C.W. in Charambrus and Inglett, "Quality of Foods and Beverages", Academic Press, New York) notes when lipid-water interaction is assisted by natural surfactant properties that viscosity of food mixes is decreased. Hulton instances the effects of soybean flour, known to be a prime source of lecithin.

An emulsifying agent which mobilizes lipids and fatty acids would in most cases act against adhesive and cohesive forces. A redispersion of flocculated colloidal matter within the plaque would be expected if lecithin were increased in quantity in the plaque. Therefore the formulation of the present invention preferably contains lecithin in a high concentration to loosen plaque on surfaces. The possible Calcium bridges, if such were a factor, would be adversely affected by fatty acids carried there in an emulsified form by lecithin. Calcium-fatty acid compounds are highly insoluble and such "free" calcium would be removed.

Thus the novel formulations preferably contain lecithin and fatty acids such as myristic acid.

Essential oils are the products of extraction from varieties of flora. Again these are complicated mixtures of organic compounds such as esters, aldehydes, alcohols, ketones and terpenes. Essential oils promote salivary flow, and act as carminatives - soothing to gastric discomfort. Most of the essential oils are antimicrobial. Only some are acceptable to the palate in concentrations of possible antimicrobial activity. Such include anethole, anise, almond, cajuput and those listed in Pharmocopeia (p 1011-1035). Of particular interest is the extract of mint, or Spearmint. Not only is it acceptable as a taste maker, in fact often preferred by consumers in ice cream, breath fresheners, chewing gum and such like, but also observation shows that unlike menthol, peppermint anethole and such, teeth feel cleaner after properly controlled solutions or emulsions of spearmint are used. The use of spearmint implies the necessary use of emulsifying agents such as the polysorbates.

Thus a formulation with spearmint has emulsifier other than, or as well as, lecithin.

An essential oil with non-toxic antimicrobial and salivary stimulating properties may be dispersed in appropriate bases according to the present invention by the use of lecithin, in and of itself a non-toxic and natural emulsifying agent.

Zinc salts are known to decrease mouth odours (Tonsetich, J., 1978 "Oral Malodour : an indicator of health status and oral cleanliness" 28 : 309). In social exchanges a foul breath is held to be a handicap, and part of this phenomenon is related to intra-oral stagnation of material within crevices between and around teeth. The generation therein of (a) acids is generally related to dental caries, (b) of toxic microbial wastes and enzymes to periodontal disease, and of (c) thiols or sulphur-containing compounds to bad breath. Thus a material decreasing viscosity of such areas should lead to a diminution of all these adverse phenomena. The zinc salts apparently combine with the odoriferous sulphur compounds and restrict odor. Among the most effective such compounds are the zinc salts of the fatty acids, and especially those of altered fatty acids such as zinc riccinoleate. A problem with many such active compounds is their insolubility in water. With the base material of the present invention, such a problem is obviated.

Preferably, in use, the delivery agent is contained in an aerosol-type container which is hermetically sealed, and non-contaminatable and furthermore prevents oxidation of its contents prior to use.

The delivery agent formulation can be made up in several ways. For example, the dispersing agents used such as Poloxamers and Polysorbates are chosen to be of a concentration such that when mixed with water an unstable, transient water-base emulsion is produced which will within 5-10 minutes begin separating. This emulsion is termed a hydrosol. However in usage, the desired amount is placed in water, such as in a paper cup, and immediately used. The unstable emulsion breaks up during agitation such as swishing around the mouth, and the active principles are deposited on surfaces such as cheeks, lips, tongue and teeth. This effect is hereinafter referred to a "contact transfer".

The use of aerosol-type packaging expands the range of components in the delivery agent. Within the typical aerosol can or container is the carrier base with a propellant gas and components added. These components are chosen for solubility in this base-gas mixture. Again the novel base solubilizes many compounds in degrees hithertofor unavailable. For example lecithin itself, substantially insoluble in water, is readily dispersed in a butane-glycerol-alcohol mixture. The propellant gases, such as butane, propane and the fluorocarbons thus act as part of the carrier base.

The preferred embodiments for an aerosol and a hydrosol in the simplest form is the usual small aerosol dispenser container or canister. Such container contains the desired mixture under pressure, and has sealed on the top a valve which is activated by forcing it down into the canister. When the valve is activated, the contents of the canister are violently propelled out through the valve. If the valve, by an extension tube, is connected to the base of the canister, and the canister is held upright, the

- 12 -

0180483

liquid goes up the tube and exits to the outside. Such is the case with a mouthspray. On the other hand, the assembly for a mouthrinse does not have an extension tube from valve to base of canister. This variety is used in the inverted position. The valve is downwards and the outside tube is pressed upwards, the valve activated, and the liquid which surrounds the valve is forced out by the propellant gas. This is done into a glass of water, and an unstable emulsion results.

Variations in formulations are needed for common purposes, and by adjusting the ratio's of alcohols, dispersants, active ingredients, percentage of gas, and types of gases as well as containers and nozzles, it is possible to produce from the formulation, a jet, an aerosol or an aerosol having an impact foam, with particles of varying diameter. In composition a variety of microbial agents may be used. Essential oils customarily used for flavour may be present or absent, and for example emulsifier and salivary diluters or viscosity reducing materials used. In many instances antisepsis or killing of micro-organisms is an inappropriate mechanism for oral health maintenance. Rather a delivery agent should be chosen which while pleasant to the taste has its main effects upon the habitat of the micro-organisms rather than directly upon them. Such approaches to hygiene or disease control prevent the development of chemically induced side-effects especially upon the total balance of normal oral dwelling micro-organisms. Moreover in periodontal disease, and also in normal function, the membrane around the teeth and the salivary glands release antimicrobial factors such as cellular elements and immunoglobulins and severe antiseptic attacks will denature and destroy these cells and protective factors.

Considerable attention in dental literature has been paid to remineralization phenomena. The inclusion of phosphates and fluorides in many formulations used in both humans and animals has been shown to prevent dental caries.

On the one hand such may repress the demineralization of teeth by a mass action ionic effect, or promote remineralization. The chemical equilibrium by both cases is tipped as it were towards stability of the mineral balance of teeth. In the usage of the proposed novel formulation we can use a phosphate or a fluoride for such purposes. In some jurisdictions the formulation is then referred to as a drug and requires considerable documentation. Such usage is elective, but the contact transfer proposed will enhance the transfer of such materials. A clean surface, a low viscosity liquid-base readily mixing with saliva will be by nature a better total arrangement for ion transfer. It will be possible to bring to a tooth surface, if so desired, a variety of ions or chemicals in a more active state than is possible in the traditional dentifrice.

Possible constituents which would operate as ecologic control of oral microbial activities, are as well as essential oils, extracts of various other plants which modify protein, carbohydrate or lipid interactions as does lecithin and sanguinarine extract. The sugar, Xylitol, is another example. There is an indication that Xylitol, unlike sucrose or sorbitol, does not thicken saliva. Thus Xylitol is a preferred sweetener and taste broadener.

The alteration of lecithin by extraction using ethanol allows the manipulation of emulsifying properties. The ethanol soluble fractions favour oil/water emulsification whereas the ethanol insoluble fractions favour water/oil emulsions. In the formulae used as an example both fractions are present.

By altering lecithin through hydrogenation, acetylation, sulfonation, halogenation and/or hydroxylation, the emulsifying properties (oil/water) can be altered and solubility in water may be improved. In the novel formulations the parent compound lecithin is chosen. The chemical manipulation of lecithin may improve the performance of a particular formulation; however this will be matters of degree rather than matters of principle.

An example of a delivery agent where the antimicrobial effect of spearmint oil is emphasized is as follows:

|  | % BY WEIGHT |
|---|---|
| Ethanol | 43.07 |
| Glycerol | 16.18 |
| Xylitol | 10.71 |
| Water | 10.71 |
| Lecithin | 5.52 |
| Polysorbate (Tween 20) | 4.59 |
| Spearmint Oil | 4.97 |
| Poloxamer (Pluronic L31) | 3.28 |
| Almond Oil | 0.97 |
|  | 100.00 |

This basic formula can be altered to give lower weights of spearmint oil without altering its wetting behaviour as general properties.

This delivery agent may be used in 0.5 ml or less quantities for immediate usage in water, or placed in an aerosol can with 30% by weight of iso-butane to produce a foam or impact foam. Higher gas ratios (50%) or different nozzles on the can will produce an impact foam or an aerosol.

Another preferred formulation includes the fatty acid, myristic acid:

|  | % BY WEIGHT |
|---|---|
| Ethanol | 40.88 |
| Glycerol | 15.35 |
| Xylitol | 10.17 |
| Water | 10.17 |
| Lecithin | 5.24 |
| Myristic Acid | 5.08 |
| Tween 80 | 4.35 |
| Spearmint Oil | 4.72 |
| Pluronic L31 | 3.11 |
| Almond Oil | .93 |
|  | 100.00 |

In the novel formulations a wide range of ingredients may be used, as the following shows:

Weight in grams used to make 100 ml

| | |
|---|---|
| Propylene Glycol | 0-91.2 |
| Xylitol | 0-15 |
| Sorbitol | 0-15 |
| Ethanol 95% | 0-78.4 |
| 70% | 0-2.2 |
| Lecithin | 0-10 |
| Silica | 0-5 |
| Poloxamer | |
| Pluronic L31 | 0-3.5 |
| L43 | 0-2.1 |
| L62D | 0-3.1 |
| Polysorbate (Tween 80) | 0-5 |
| Flavour and active oil | |
| $\ell$-carvone ($\ell$-spearmint oil) | 2.4-4.8 |
| Spearmint Oil (natural extract) | 0-4.6 |
| Almond Oil | 0-0.91 |
| Glycerol | 0-88.3 |
| Water | 0-61.0 |
| Dipropylene Glycol | 0-5.1 |

The role of various ingredients and their possible alternatives is described as follows. The base can be any mixture of polyhydric alcohols with minor additions of water. Many varieties of sweeteners or flavour extenders can be used, such as sorbitol, mannitol or xylitol. Lecithin or its derivatives (ethoxylated, hydroxylated and so on) is used as a natural surfactant or oil-water interfacial agent. Other agents such as sorbitan and ethoxylated sorbitan esters may be used in whole or in part for the surfactant. The use of Poloxamer (Pluronics) and the polysorbates is related to emulsification both in the container and in the mouth or in the prior vehicle in the case of a mouthwash. Others have observed in industrial practice that successful working emulsions may be made by using two materials for emulsification. The choice are two such agents - one with a low HLB value, and one with a high HLB. The HLB value refers to the hydrophylic-lipophylic balance. A low HLB value favours water/oil emulsions, a high one favours oil/water emulsions. In a mixture with little water such as the novel formulation range, the water/oil emulsion probably occurs. However the presence of a high HLB value agent, will produce instability if such a mixture is quickly diluted in water. The rapid agitation of this produces what we have called "contact transfer". The small colloidal particle or bubble, when contacting a surface, will tend to wet that surface whether it is lipophylic or hydrophlic when the colloidal particle has both low and high HLB value surfactants therein.

As previously indicated, apart from the delivery agent itself, a variety of other factors determine the physical nature of the expelled fluids. The percentage of gas by weight in the tube has effects; the more the gas, the finer the particles (within limits). If the gas chosen has a higher static pressure (butane is about 17 psi and propane about 100 psi), again the form of droplets is altered. The nozzle and its distance from the valve are significant, and by these variations foams or fine sprays can be created.

In a preferred embodiment, 10 ml cans with miniaturized valves common to the commercial mouth freshener cans is used with dip-tube (oral spray), without dip-tube mouthrinse), and with a standard aerosol cap. A 30 to 50% by weight of gas (butane plus propane to give 45 psi) is preferably used. The delivery agents described herein give an active spray for intra-oral use and an active contact transfer mouthrinse.

The viscosity of the delivery agent and the solubility of the preferred gas mixture have some influence upon the spray form. For example, a preferred delivery agent uses a glycerol-ethanol mix which is used at 46 psi. It is found that such a mix suspends or dissolves the other components, dissolves sufficient gas to help in the solubilization of the added components, and is of a viscosity that on discharge through a nozzle produces a spray at 10 to 15 cms distance.

In aerosol technology there is still a need to use practical experiments rather than existing theory or empirical knowledge, to find out whether a mixture of alcohols and additives at a particular pressure with a particular gas will discharge as an aerosol with suitable drop size or will be a foam or contact foam.

To demonstrate in laboratory conditions the basis for the oral cleaning observed, a series of experiments were done.

The experiments on wetting utilize glass slides, saliva and water, with various forms of treatment. The rationale for such experiments is that human enamel and/or dentin is seldom exposed in the mouth. The working surface of a tooth is not mineral, except when highly polished and outside the mouth. Glantz and others (Frank and Leach 1981) state "all these surfaces (referring to human enamel, gold, dental polymer and stainless steel) of extremely different original chemistry are being brought to the same surface by a (intra-oral) mechanism of surface film adsorption". Thus the intra-oral film is the "surface" of the tooth. Moreover wetting is markedly influenced by the topography of the surface. Thus to be preferred in experiments where saliva can be shown to have a wide range of effects (due to diet, action of spitting, relative salivary flow etc.), a variation due to topography is unacceptable. Thus all experiments show the effects upon saliva itself, or upon salivary interaction with a smooth clean surface not unlike dental enamel. The contact angle for water on plane ground but otherwise untreated human enamel is 49.81 (s.e. 0.12 n=390) according to Glantz (1969 "Wetability and Adhesiveness", Odont Revy 20: Supl. 17). The glass slides and apparatus gave values of 53.97° (s.e. 4.03, n=6) this lesser accuracy reflects, amongst other things, uncontrolled humidity. However as the following results are comparing contact angles for 1 to 57°, it is within simple experimental error to suggest that glass is suitable surface to test the film forming properties of saliva. Each test method is but an attempt to quantify effects which are quite apparent to anyone using these materials in the mouth.

CONTACT ANGLE MEASUREMENTS

A glass-slide was cleaned with isopropyl alcohol. Various formulations were used to test the wetting capacity of saliva and water upon this glass surface. There is a similarity between glass and dental enamel surfaces in that both are low energy surfaces, and that saliva will coat such surfaces equally.

The test was conducted in a Toolmakers microscope (Olympus STM) lying on its side. A holding jig placed the glass slide directly in the beam of the microscope, which is by virtue of the construction at the centre of the rotating stage. The stage can be rotated, and the outside is calibrated so that angles may be measured in increments of 2 minutes of a degree.

The glass slide was sprayed with an aerosol and the remains after the solvent had evaporated was spread on the slide. A drop of saliva from volunteers was placed on the slide and the drop positioned in the device so its profile was silhouetted in the illuminating beam. The contact angle between drop and glass was readily visible at a magnification of about 50X.

The glass slide was horizontal. The eyepiece was rotated so that the cross-hair was positioned as a tangent to curved meniscus profile. The angle on the rotating stage was noted. The stage was then rotated such that the edge of the glass slide was coincident with the cross-hair, and the angle was read again; the difference between the two readings is the estimated contact angle, as shown in Tables 1, 2 and 3.

In Table 1, a novel specimen delivery agent (D.A.) is contrasted with a commercial Breath Freshener (70% liquids – 30% butane aerosol), and with ethanol (95% with water; 70% liquid – 30% butane aerosol). The wetting characteristics of ethanol, a very well known biologic cleanser used in skin sterilization and cleaning, is matched by the SKOTT formulation. Generally a mouthwash or spray of ethanol or even flavoured ethanol at a concentration of 95% would be regarded as impractical for reasons associated with possible alcohol intoxications and social implications. The inclusion of other ingredients in the commercial breath freshener impedes the alcohol wetting characteristics markedly. There is a clear difference (statistically significant) between D.A. and the Commerical Breath Freshener in wetting capacity.

### TABLE 1

Contact angle measurements of saliva

and water upon treated glass slides; in degrees;

| Volunteer | D.A. | | BF | | ETHANOL | |
| | Saliva | Water | Saliva | Water | Saliva | Water |
| --- | --- | --- | --- | --- | --- | --- |
| 1 | 2.93 | 2.27 | 5.53 | 19.37 | 5.33 | 1.20 |
| 2 | 2.67 | 5.03 | 15.30 | 31.67 | 5.23 | 2.00 |
| 3 | 4.33 | 4.12 | 13.53 | 29.85 | 2.10 | 2.60 |
| 4 | 4.17 | 4.65 | 22.43 | 27.93 | 5.62 | 5.33 |
| 5 | 2.12 | 5.58 | 25.40 | 30.53 | 1.50 | 3.63 |
| Mean x | 3.24 | 4.33 | 16.44 | 27.87 | 3.96 | 2.95 |
| s.d. | 0.97 | 1.27 | 7.83 | 4.94 | 1.98 | 1.60 |

D.A.        is a fine dispersion contact transfer delivery agent
BF          is a commercial breath freshener
ETHANOL     is 95% ethyl alcohol in an aerosol container with 30% butane

0180483

## TABLE 2

Contact angles in degrees of saliva
and water on treated glass; numbers of
determinations when more than 1 in brackets.

### Single Ingredients

| Variant | Saliva | Water | Variant | Saliva | Water |
|---------|--------|-------|---------|--------|-------|
| D.A. 0 | 33.63 (3) | 38.94 (2) | D.A. S (3) | 9.93 | 6.45 |
| D.A. L | 5.30 | 2.55 | D.A. P | 16.00 | 17.43 |
| D.A. T | 6.68 | 2.25 | | | |

### Two Ingredients

| Variant | Saliva | Water | Variant | Saliva | Water |
|---------|--------|-------|---------|--------|-------|
| D.A. PT | 4.31 (3) | 3.00 (3) | D.A. LS | 9.03 | 4.92 |

### Three Ingredients

| Variant | Saliva | Water | Variant | Saliva | Water |
|---------|--------|-------|---------|--------|-------|
| D.A. PTL | 2.56 (2) | 4.46 (2) | D.A. SPT | 3.07 | 5.53 |

D.A. 0 is an alcohol-glycerol mixture (81:19 proportion)

| | |
|---|---|
| S | is D.A. 0 with 7% by volume spearmint oil |
| L | is D.A. 0 with 7% by volume lecithin |
| P | is D.A. 0 with 5% by volume Pluronic* L31 |
| T | is D.A. 0 with 6% by volume Tween* 80 |
| PT | is D.A. 0 with 6% by volume Tween 80 and 4% of Pluronic L31 |
| LS | is D.A. 0 with 7% by volume Lecithin and 7% spearmint oil |
| SPT | is D.A. 0 with 6% by volume Tween 80, 4% Pluronic L31 and 5% spearmint oil |
| PTL | is D.A. 0 with 6% by volume Tween 80, 4% Pluronic L31 and 7% Lecithin |

* Trade Mark

0180483

## TABLE 3

Contact angles (in degrees) of saliva and water
on Treated glass; from 3 subjects, using four formulas

| Code | Saliva | Water | Code | Saliva | Water |
|------|--------|-------|------|--------|-------|
| SPT | 3.12,7.17 3.07 | 2.00,14.40 5.53 | SPTL | 7.47,10.40 8.95 | 6.13,4.77 3.63 |
| PT | 2.10,10.93 4.31 | 2.10, 5.33 5.63 | PTL | 6.50,7.00 2.56 | 5.35,3.58 4.46 |

See Previous Code.

## TABLE 4

Contact Angles of Saliva and water on treated
glass, using base of ethanol and glycerol (D.A. 0)

| Additive | Note | Saliva | Water |
|----------|------|--------|-------|
| Cinnamon Oil | Essential Oil | 3.57 | 11.33 |
| Eucalyptol | Essential Oil | 33.17 | 18.07 |
| Oil of Wintergreen | Essential Oil | 53.42 | 57.50 |
| Menthol | Essential Oil | 4.57 | 9.92 |
| Anise seed oil | Fixed Oil | 38.07 | 47.97 |

In Table 2, the functions of the ingredients are shown. The Poloxamer (Pluronic L31) by itself is mildly effective (see entry D.A. P) whereas D.A. S (spearmint oil) demonstrates wetting action of some essential oils. The surfactants lecithin (D.A. L) and polysorbate (Tween 80 or T) are active in the base carrier. As the ingredients are blended, the maintenance of wetting action is preserved (entries under Two and Three Ingredients).

Further detail emerges in Table 3. The saliva of three subjects was used, with basic mixtures with and without lecithin and spearmint oil. An Analysis of Variance (ANOVA) was done for Main Effects. The variability in human saliva was shown to be significant at 0.99 probability. The inclusion of spearmint oil was significant at 10-15% level.

In Table 4, six percent of other flavouring oils was compared to spearmint, in the carrier base D.A. O. Cinnamon and menthol are similar in effects.

Subjective analysis must follow the quantitation. Lecithin does not in itself appear to operate in a similar fashion for all people tested. However that is difficult to quantitate is that lecithin appears to operate on cheeks, lips and tongue rather than teeth. Lecithin in effect is thus on the soft tissues. Choice of essential oils is restricted by common practice and general population behaviour. Cinnamon is not universally liked. The amount of menthol used is beyond the level acceptable to most users. Spearmint, or peppermint is the preferred essential oil because it is acceptable, popular and because its intensity of flavour can be masked by sweeteners.

ALTERATIONS IN COHESION AND ADHESION

The alteration of surface wetting actions, and thus the clinging nature of saliva is one basis of this invention. Simple tests may be used to illustrate the disruption of the molecular forces within the saliva.

These molecular forces cause adherence (tackiness) and coherence (viscosity) of the saliva which leads to the retention of food in, and around teeth.

The cohesion of the long molecules within saliva, and in other biological secretions, causes the thick liquids to form ropes as some fluid is drawn out. Spinnbarkeit is the phenomenon where a viscid liquid can be drawn out as a rope. Such was described clearly by Dewar and Parfett (J.D. Research 33:356 October 1954). Since that time, it has been suggested that saliva in its rheological properties (texture, viscosity, spinnbarkeit) is temperature sensitive. We have altered the test described by conducting, wherever possible, all measurements on saliva held at 35°C.

A glass rod was sucked by a volunteer for 30 seconds or more. Then he deposited about 2 ml of saliva upon a glass slide. Both were at 37°C before use. The rod was placed vertically in a testing machine (MTS) and the slide upon a hot plate directly below the rod. The hot plate created a 37°C temperature upon the glass slide. The rod was lowered onto the surface of the saliva sample. Then the rod was withdrawn at a rate of 70 mm/min. As soon as the rope thus drawn up broke, the distance between rod end and the saliva "bubble" was measured - giving a value for Spinnbarkeit in millimeters.

To test formulations, the rod was sprayed at a distance of 20-30 cms after the rod had been sucked and thus covered with salivary film. The rod was end-on to the spray. During the experiment with each volunteer saliva samples were done to check that their saliva was not changing markedly.

TABLE 5

Test for ropiness - Spinnbarkeit; in mms

| Volunteer | 1 Saliva | 2 D.A. | 3 Saliva | 4 BF | 5 Eth | 6 Saliva |
|---|---|---|---|---|---|---|
| 1 | 12,8,4 | 3,3,3 | 8 | 4,10,6 | 8,5,8 | 11,7,3 |
| 2 | 11,8,16 | 6,7,5 | 10 | 10,8,8 | 10,4,6 | 8,4,6 |
| 3 | 6,4,6 | 6,6,6 | 6 | 9,6,5 | 4,6,7 | 9,4,4 |
| 4 | 8,8,11 | 6,2,4 | 6,10,8 | 7,8,11 | 10,10,10 | 6,9,7 |
| Mean | 8.5 | 4.9 | 8.0 | 7.7 | 7.3 | 6.5 |
| s.d. | 3.5 | 1.7 | 1.8 | 2.2 | 2.4 | 2.5 |

The results are shown in Table 5. The row across for each volunteer donor is serially placed; Column 1 precedes 2 in time, 2 precedes 3 and so on. As time wears on in each series, the saliva thins. The test materials were the novel formulation- D.A., BF or a common commercial breath freshener, and Eth-95% ethyl alcohol-water mix. All these were used as aerosols with 30% gas. A glance across the columns show that D.A. reduces spinnbarkeit, while the others have no effect, except perhaps to increase the ropiness.

Thus is demonstrated that the cohesion of the saliva is decreased by a simple spray.

As an example of Contact Transfer, the test for Spinnbarkeit was repeated but the glass rod, having been coated with saliva by the volunteer, was stirred for 10 seconds in water, in a commercial mouth wash and in 2 formulations of the invention. The formulations D.A. 21,24 were in aerosol cans and a 1-2 second discharge of formulation was made underneath (and thus into) the water. The results are shown in Table 6.

An Analysis of Variance was performed. Under Main Effects the D.A. formulations were significantly (5%) different to both mouthwash and water. There was a small difference between water and the mouthwash. The means and the significant difference between 2 means from the "Student" t-test was calculated. This shows clearly that the D.A. Contact Transfer mode is different to mouthwash and/or water - as high as a 60% difference.

## TABLE 6

Contact Transfer: Spinnbarkeit after saliva coated rod swirled in test material: in mm.

| Volunteer | Water | Mouthwash | D.A. 21 | D.A. 24 |
|---|---|---|---|---|
| 1 | 7,9,5 | 4,13,4 | 4,6,4 | 3,2,2 |
| 2 | 7,6,6 | 3,6,6 | 6,3,3 | 3,4,4 |
| 3 | 8,6,8 | 3,4,2 | 4,5,2 | 5,5,5 |
| Mean | 6.9 | 5.0 | 4.1 | 3.7 |
| t difference | 0.8 | 0.8 | 0.8 | 0.8 |
| Mean | | 5.9 | | 3.8 |
| t difference | | 0.7 | | 0.7 |

The intra-oral effects of the novel formulations are partially shown by the preceding experiments upon tackiness and viscosity. The role of the components is related to the subsequent effects as felt within the mouth.

Finally, it should be noted that the quality and nature of saliva changes during the day in relation to many physiologic, and indeed even psychologic stimuli. Wherever possible test samples have not been allocated in the same order of test (Table 6) or the immediate state of the saliva has been retested during the experiment. Thus, in Table 5, for example, columns 4 and 5 should be contrasted with columns 3 and 6 rather than with column 1, and likewise column 2 with columns 1 and 3. On this basis D.A. shows a 60% decrease or 4.9/8.25, and the BF and ethanol show no difference, 7.3/7.25.

CLAIMS

1. A pressurized delivery agent for oral hygiene which comprises a potable mono-, di- or polyhydric alcohol or mixture thereof as non-aqueous carrier base, and a substantially water-insoluble surfactant, the agent being in the form of a stable solution or suspension which, when released from pressure and mixed with air or water, forms an unstable aerosol or hydrosol respectively which then achieves a more stable state by coating out on a surface with which it contacts.

2. A pressurized delivery agent as claimed in claim 1 which further comprises a propellant gas which is a low vapour pressure hydrocarbon or fluorocarbon, nitrous oxide or carbon dioxide and which increases the solubility of the carrier base for the active components of the delivery agent.

3. A pressurized delivery agent as claimed in claim 1 or claim 2 which further comprises lecithin or a derivative thereof as the substantially water-insoluble surfactant.

4. A pressurized delivery agent as claimed in any one of the preceding claims which further comprises a fatty acid which is stearic, oleic, palmitic or myristic acid.

5. A delivery agent as claimed in any one of the preceding claims wherein the substantially water-insoluble surfactant is a naturally occurring surfactant assisted by a potable surfactant which is a poloxamer or a polysorbate.

6.    A delivery agent as claimed in any one of the preceding claims further comprising a water-soluble or water-insoluble zinc salt which decreases the vaporization of odoriferous materials.

7.    A delivery agent as claimed in any one of the preceding claims further comprising a fluoride or phosphate salt which aids integrity and enhances the resistance of dental enamel.

8.    A pressurized delivery agent for oral hygiene, which comprises a stable solution or suspension of one or more substantially water-insoluble active components in a potable polyhydric alcohol with lecithin or a derivative thereof, the stable solution or suspension being capable of forming an unstable aerosol or hydrosol when contacted with air or water respectively from which the active component coats onto a surface with which the unstable aerosol or hydrosol comes into contact.

9.    A delivery agent as claimed in any one of the preceding claims wherein the said potable alcohol is glycerol, propylene glycol, dipropylene glycol, ethylene propylene glycol or ethanol, or mixtures thereof.